# EUROPEAN PATENT APPLICATION

(11) **EP 3 521 827 A1**
(43) Date of publication of application: **07.08.2019**
(21) Application number: 17858248.2
(22) Date of filing: 26.09.2017
(51) Int. Cl.: G01N 33/68, C12Q 1/68, G01N 33/15, G01N 33/493, G01N 33/50, G01N 33/53

(54) **INSPECTION METHOD ENABLING SPECIFIC DIAGNOSIS OF PATHOLOGICAL STATE OF DIABETIC NEPHROPATHY AT EARLY STAGE**

(30) Priority: 03.10.2016 JP 2016195420
(71) Applicant: HuBit genomix, Inc., Chuo-ku Tokyo 104-0045 (JP); Fuso Pharmaceutical Industries, Ltd., Osaka-shi, Osaka 541-0045 (JP)
(72) Inventor: DOI, Toshio, Kyoto-shi Kyoto 602-0946 (JP); TOMINAGA, Tatsuya, Tokushima-shi Tokushima 779-3124 (JP); ICHIEN, Go, Tokyo 104-0045 (JP); YAMAMOTO, Keiichi, Osaka-shi Osaka 536-8523 (JP)
(74) Representative: J A Kemp
(86) International application number: PCT/JP2017/034699
(87) International publication number: WO 2018/066409

(57) **Abstract**

The present invention provides a test method which enables specific diagnosis of early-stage pathology of diabetic nephropathy. There is provided a method of detecting onset or a risk of onset of diabetic nephropathy, comprising measuring IgG4 in a biological sample derived from a subject. This method may further comprise measuring Smad1 in the biological sample derived from the subject. Preferably, both IgG4 and Smad1 are measured.

## Description

### TECHNICAL FIELD

The present invention relates to a test method which enables specific diagnosis of early-stage pathology of diabetic nephropathy.

### BACKGROUND ART

Diabetic nephropathy (DN) is the major cause of chronic kidney disease (CKD) and the most common causative disease of end-stage renal disease (ESRD)^{1,2} throughout the world (Non-Patent Documents Nos. 1 and 2). In the natural history of diabetic nephropathy, clinical manifestations are not observed for a long period, but renal lesions thereof may potentially exist and progress before increase in albuminuria (AER) and decrease in glomerular filtration rate (GFR) become detectable (Non-Patent Document No. 3)³. Once diabetic nephropathy has progressed into overt nephropathy with positive urine protein, it is very difficult to prevent the progression into end-stage renal failure. Therefore, it is essential to grasp nephropathy before it is diagnosed overt nephropathy. As characteristic findings of diabetic nephropathy, thickening of the glomerular basement membrane (GBM) and expansion of the mesangial matrix are enumerated. In particular, expansion of the mesangial matrix and subsequent structural changes in the glomeruli (such as decrease in the filtration surface) are correlated with increase in albuminuria and decrease in GFR. The rate of mesangial matrix hyperplasia in the glomeruli is critical as a predictor for change (decrease) in renal function (Non-Patent Documents Nos. 4 and 5)^{4,5}. Although persistent presence of microalbuminuria (MA) is regarded at present as the best early-stage biomarker for DN risk, it is believed that histological lesions in type I and type II diabetes have already progressed before microalbuminuria occurs. In brief, mesangial matrix hyperplasia is recognized occasionally even in patients with normal albuminuria, and it is impossible to grasp mesangial matrix hyperplasia and GBM thickening by means of microalbuminuria. Further, approximately 35-45% of the type I and type II diabetes cases where microalbuminuria is observed will progress to overt nephropathy in the subsequent 6 or 7 years (Non-Patent Document 6)⁶. On the other hand, one third thereof will spontaneously revert to normal albuminuria. Therefore, albuminuria is not specific to the progression of nephropathy, making it difficult to diagnose early-stage nephropathy by means of microalbuminuria. Early-stage nephropathy exists considerably earlier than the time when overt nephropathy develops. To reveal specific biomarkers that precisely reflect early-stage nephropathy is the only way which enables diagnosis and treatment of diabetic nephropathy.

Therefore, search for biomarkers other than urinary albumin has been carried out for more than 20 years. Not only measurement of urinary type IV collagen [Motohide Isono, Masakazu Haneda, Masaki Togawa, Tsutomu Shikano, Shinnichi Araki, Takahiko Nakagawa, Takeru Ishida, Ken Inoki, Kenichi Obata and Ryuichi Kikkawa, Analysis of Urinary Type IV Collagen in NIDDM Subjects as a Marker for Diabetic Nephropathy, J. Japan Diab. Soci. 1996; 39: 599-604 (Non-Patent Document No. 7); Yasuhiko Ieki and Eisuke Takazakura, Significance of Measurement of Urinary Type IV Collagen as a Predictor of Progress of Early-Stage Diabetic Nephropathy, J. Japan Diab. Soci. 1999; 42: 859-862 (Non-Patent Document No. 8)], but also measurement of total urinary IgG (not urinary IgG4) excretion have been suggested useful (based on the results described below) as indicators for early diagnosis of diabetic nephropathy.

Negishi et al. have compared urinary IgG, IgG4 and albumin excretion in 61 non-insulin-dependent diabetes mellitus (NIDDM) patients and 36 healthy controls, examined the clinical significance thereof in diabetic nephropathy, and obtained the following findings. 1) Total urinary IgG index was significantly high (p<0.01) in normoalbuminuria group compared to control group; and was significantly high (p<0.01) in microalbuminuria group compared to normoalbuminuria group. 2) Urinary IgG4 index was almost equal in normoalbuminuria group and control group; no difference was observed between these two groups. However, urinary IgG4 index was significantly high (p<0.01) in microalbuminuria group compared to normoalbuminuria group. (Kiyohiko Negishi, Satomi Shibasaki, Yasuaki Ishimaru et al., Total Urinary IgG and IgG4 Excretion in Non-Insulin-Dependent Diabetes Mellitus - Using a Sensitive Total IgG and IgG4 Assay -, J. Japan Diab. Soc. 1992; 35: 1007-1011 (Non-Patent Document No. 9)).

Yashima et al. compared urinary IgG, IgG4, albumin and transferrin excretions, urinary β2 microglobulin level and urinary NAG activity with glomerular lesions in 197 NIDDM patients, and examined whether or not measurement of urinary IgG excretion is useful as an indicator for disorder in the glomerular basement membrane before the occurrence of microalbuminuria. As a result, Yashima et al. obtained the following findings. 1) While total urinary IgG excretion increased from the normoalbuminuria stage, urinary IgG4 excretion did not. 2) Although total urinary IgG excretion at the normoalbuminuria stage increased significantly keeping pace with the progression of glomerular diffuse lesions, no relation was recognized between urinary albumin excretion and the progression of glomerular diffuse lesions (Isao Yashima, Toshihide Hirayama, Hideo Shiiki, Masao Kanauchi and Kazuhiro Dohi. Diagnostic significance of urinary immunoglobulin G in diabetic nephropathy. Jpn J Nephrol 1999; 41: 787-796 (Non-Patent Document No. 10)).

### PRIOR ART LITERATURE

### Non-Patent Documents

Non-Patent Document No. 1: Krolewski M, Eggers PW, Warram JH. Magnitude of end-stage renal disease in IDDM: a 35 year follow-up study. Kidney Int 1996; 50:2041-2046.
Non-Patent Document No. 2: Bojestig M, Amqvist HJ, Hermansson G, Karlberg BE, Ludvigsson J. Declining incidence of nephropathy in insulin-dependent diabetes mellitus. N Engl J Med 1994; 330:15-18.
Non-Patent Document No. 3: Parving H-H, Mauer M, Ritz E. Diabetic nephropathy. In: Brenner BM, ed. Brenner & Rector's The Kidney. 7th ed. Philadelphia, USA. Saunders, Elsevier's Health Sciences Department, 2004: 1777-1818.
Non-Patent Document No. 4: Mauer SM, Steffes MW, Ellis EN, Sutherland DE, Brown DM, Goetz FC. Structural-functional relationships in diabetic nephropathy. J Clin Invest 1984; 74:1143-1155.
Non-Patent Document No. 5: Kriz W, Lehir M. Pathways to nephron loss starting from glomerular disease- Insights from animal models. Kidney Int 2005; 67:404-419.
Non-Patent Document No. 6: Caramori ML, Kim Y, Huang C, et al. Cellular basis of diabetic nephropathy: 1. Study design and renal structural-functional relationships in patients with long-standing type 1 diabetes. Diabetes 2002; 51:506-13.
Non-Patent Document No. 7: Motohide Isono, Masakazu Haneda, Masaki Togawa, Tsutomu Shikano, Shinnichi Araki, Takahiko Nakagawa, Takeru Ishida, Ken Inoki, Kenichi Obata and Ryuichi Kikkawa, Analysis of Urinary Type IV Collagen in NIDDM Subjects as a Marker for Diabetic Nephropathy, J. Japan Diab. Soci. 1996; 39:599-604
Non-Patent Document No. 8: Yasuhiko Ieki and Eisuke Takazakura, Significance of Measurement of Urinary Type IV Collagen as a Predictor of Progress of Early-Stage Diabetic Nephropathy, J. Japan Diab. Soci. 1999; 42:859-862
Non-Patent Document No. 9: Kiyohiko Negishi, Satomi Shibasaki, Yasuaki Ishimaru et al., Total Urinary IgG and IgG4 Excretion in Non-Insulin-Dependent Diabetes Mellitus - Using a Sensitive Total IgG and IgG4 Assay -, J. Japan Diab. Soc. 1992; 35:1007-1011
Non-Patent Document No. 10: Isao Yashima, Toshihide Hirayama, Hideo Shiiki, Masao Kanauchi and Kazuhiro Dohi. Diagnostic significance of urinary immunoglobulin G in diabetic nephropathy. Jpn J Nephrol 1999; 41:787-96

### DISCLOSURE OF THE INVENTION

### PROBLEM FOR SOLUTION BY THE INVENTION

The present invention aims at providing a test method which enables specific diagnosis of early-stage pathology of diabetic nephropathy.

### MEANS TO SOLVE THE PROBLEM

The present inventors have proved that Smad1 (a molecule functioning downstream of the TGF-β superfamily receptors) is an important transcription factor for the α1 and α2 chains of type IV collagen, which are major components of mesangial matrix expansion observed in diabetic nephropathy^{7,8}. Smad1 is the most important factor for the expansion of the mesangial matrix in STZ (streptozotocin)-induced diabetic rats, and the mesangial expansion was strongly correlated not with albuminuria but with urinary Smad1⁹. Further, the present inventors have confirmed that early increases in urinary Smad1 level predict later development of mesangial matrix expansion in both STZ rats and db/db mice (non-insulin-dependent (type 2) diabetes model animal)¹⁰. The present inventors have demonstrated in various studies that Smad1 signaling pathway is strongly associated with the pathogenesis in diabetes animal models¹¹⁻¹⁷.

In diabetic nephropathy, loss of charge selectivity in the glomeruli occurs prior to decrease in glomerular filtration function¹⁸. It was confirmed that IgG4, being charged negatively, selectively bound in a linear pattern to the GBM of early-stage and overt nephropathy¹⁹. Based on these findings, the present inventors have hypothesized that not total urinary IgG (which is suggested to be an early diagnosis indicator for DN) but urinary IgG4 excretion is strongly associated with functional and pathological problems of the GBM. The hypothesis suggests that urinary IgG4 may be a useful biomarker for detecting lesions in the GBM. In this study, the present inventors have elaborated a highly sensitive and specific measuring method for detecting urinary IgG4.

In this study, the present inventors have revealed that both urinary IgG4 and urinary Smad1 are specific biomarkers for GBM lesions and mesangial matrix expansion (which are pathological findings characteristic of diabetic early-stage nephropathy) and that these factors are predictors for overt nephropathy in diabetes.

The gist of the present invention is as described below.
(1) A method of detecting onset or a risk of onset of diabetic nephropathy, comprising measuring IgG4 in a biological sample derived from a subject.
(2) The method of (1) above, further comprising measuring Smad1 in the biological sample derived from the subject.
(3) The method of (1) or (2) above, wherein the biological sample is urine.
(4) The method of any one of (1) to (3) above, which is used for early diagnosis of diabetic nephropathy.
(5) A kit for detecting onset or a risk of onset of diabetic nephropathy, comprising a reagent(s) for measuring IgG4.
(6) The kit of (5) above, further comprising a reagent(s) for measuring Smad1.
(7) A method of diagnosing diabetic nephropathy in a subject, comprising:
   a. obtaining a biological sample from the subject,
   b. measuring IgG4 level in the biological sample from the subject, and
   c. judging that the subject has developed diabetic nephropathy or has a high risk of developing diabetic nephropathy based on the IgG4 level measured.
(8) A method of diagnosis and treatment of diabetic nephropathy in a subject, comprising:
   a. obtaining a biological sample from the subject,
   b. measuring IgG4 level in the biological sample from the subject,
   c. judging that the subject has developed diabetic nephropathy or has a high risk of developing diabetic nephropathy based on the IgG4 level measured, and
   d. starting treatment of diabetic nephropathy in the subject who is judged as having developed diabetic nephropathy or as having a high risk of developing diabetic nephropathy.
(9) The method of (7) or (8) above, wherein the subject is judged as having developed diabetic nephropathy or as having a high risk of developing diabetic nephropathy when the IgG4 level in the biological sample from the subject is higher than the IgG4 level in a corresponding biological sample from healthy persons who have not developed diabetic nephropathy.
(10) The method of (7) or (8) above, wherein the subject is judged as having developed diabetic nephropathy or as having a high risk of developing diabetic nephropathy when the IgG4 level in the biological sample from the subject is higher than a cutoff value.
(11) The method of (7) or (8) above, wherein the biological sample from the subject is urine and the subject is judged as having developed diabetic nephropathy or as having a high risk of developing diabetic nephropathy when the IgG4 level in the subject's urine is 39.2 µg/g Cr or more.
(12) The method of (7) or (8) above, further comprising measuring Smad1 level in the biological sample from the subject and judging that the subject has developed diabetic nephropathy or has a high risk of developing diabetic nephropathy based on the Smad1 level measured.
(13) The method of (12) above, wherein the subject is judged as having developed diabetic nephropathy or as having a high risk of developing diabetic nephropathy when the Smad1 level in the biological sample from the subject is higher than the Smad1 level in a corresponding biological sample from healthy persons who have not developed diabetic nephropathy.
(14) The method of (12) above, wherein the subject is judged as having developed diabetic nephropathy or as having a high risk of developing diabetic nephropathy when the Smad1 level in the biological sample from the subject is higher than a cutoff value.
(15) The method of (14) above, wherein the biological sample from the subject is urine and the subject is judged as having developed diabetic nephropathy or as having a high risk of developing diabetic nephropathy when the Smad1 level in the patient's urine is 0.15 µg/gCr or more.
(16) The method of (8) above, wherein the treatment of diabetic nephropathy is glycemic control and/or antihypertensive therapy.
(17) The method of (7) or (8) above, wherein the subject is at an early stage of diabetic nephropathy.
(18) A method of identifying substances effective in preventing and/or treating diabetic nephropathy, comprising judging whether or not a test substance inhibits expression of IgG4.
(19) The method of (2) above, wherein IgG4 and Smad1 levels are measured by immunoassay.
(20) The kit of (6) above for detecting onset or a risk of onset of diabetic nephropathy by measuring urine samples.

### EFFECT OF THE INVENTION

According to the present invention, it becomes possible to predict the onset of diabetic nephropathy and to treat early-stage nephropathy. Treatment of early-stage nephropathy may potentially inhibit progression of diabetic nephropathy.

The present specification encompasses the contents disclosed in the specification and/or drawings of Japanese Patent Application No. 2016-195420 based on which the present patent application claims priority.

### BRIEF DESCRIPTION OF THE DRAWINGS

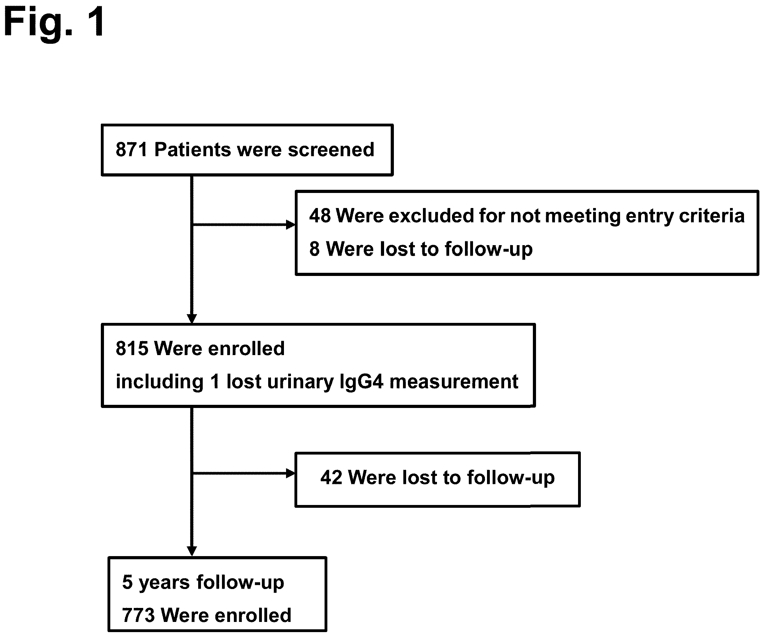

[Fig. 1] Enrollment and follow-up of subjects. For follow-up study, 871 patients with type 2 diabetes were screened and 815 patients were enrolled. Finally, at the end of 5-year follow-up, 773 patients were analyzed for eGFR test.

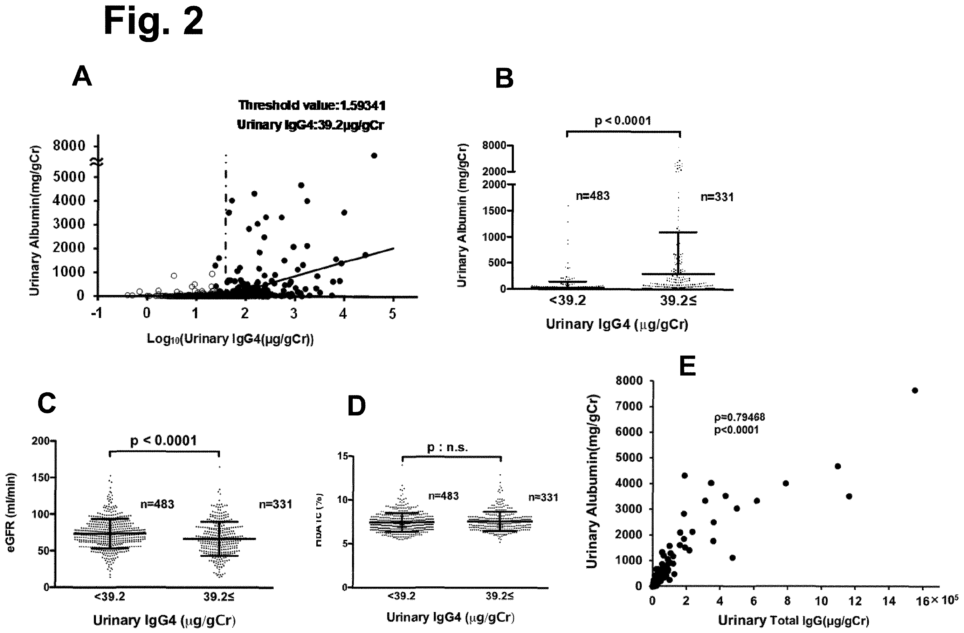

[Fig. 2] A. Correlation analyses between urinary albumin and log urinary IgG4 by hockey stick regression. Two different distributions occurred among all patients. Correlation coefficients of the group with higher urinary IgG4 were as follows. r=0.435, p<0.0001. Threshold value was 1.59341, and cutoff value was 39.2 µg/g Cr. B. The group with urinary IgG4 ≥39.2 µg/g Cr exhibits a significantly high urinary albumin value. Data are shown as mean ± standard deviation. C. The group with urinary IgG4 ≥39.2 µg/g Cr exhibits a significantly low eGFR. Data are shown as mean ± standard deviation. D. No difference is observed between the two groups with respect to HbA1c. Data are shown as mean ± standard deviation. E. Correlation analyses of urinary albumin and total urinary IgG.

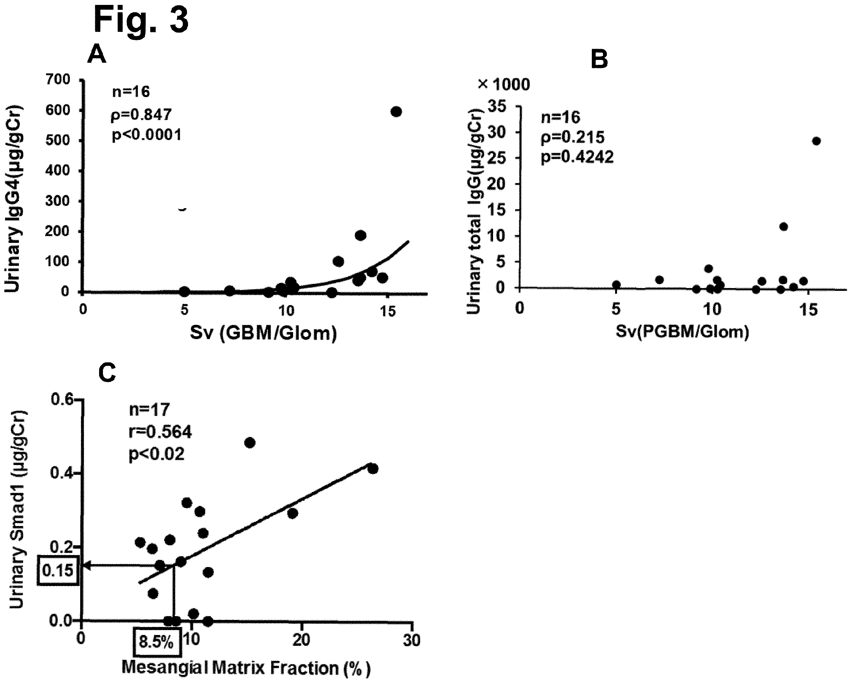

[Fig. 3] A. Correlation between urinary IgG4 and glomerular basement membrane (GBM) fraction. B. Correlation between total urinary IgG and GBM fraction. C. Correlation between urinary Smad1 and the mesangial matrix.

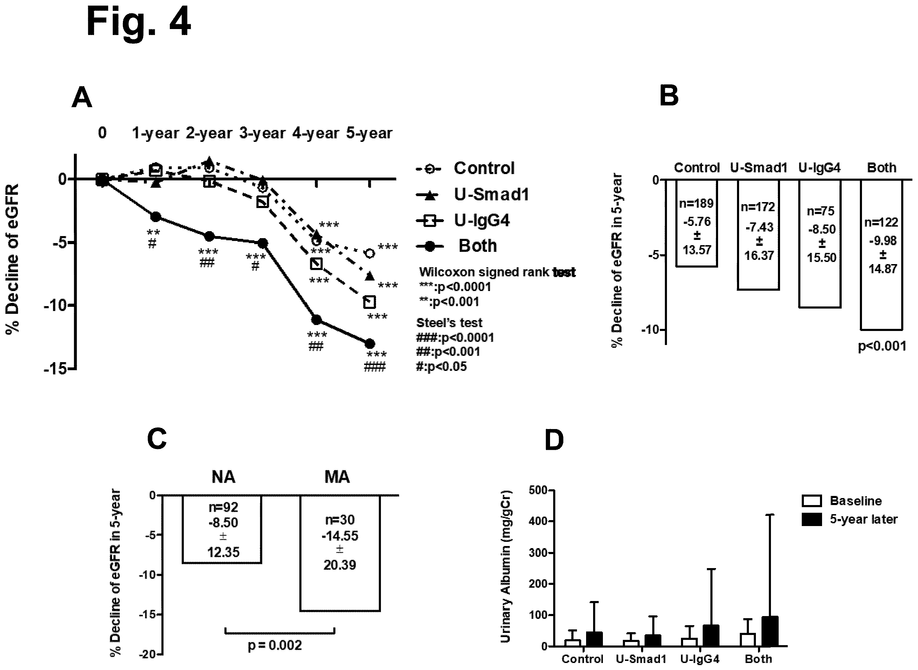

[Fig. 4] Control group [urinary Smad1 (µg/g Cr) <0.15; urinary IgG4 (µg/g Cr) <39.2 at year 0 (at the start of follow-up)], U-Smad1 group [urinary Smad1 (µg/g Cr) ≥0.15; urinary IgG4 (µg/g Cr) <39.2 at year 0 (at the start of follow-up)], U-IgG4 group [urinary Smad1 (µg/g Cr) <0.15; urinary IgG4 (µg/g Cr) ≥39.2 at year 0 (at the start of follow-up)], Both positive group [both positive at year 0 (at the start of follow-up); urinary Smad1 (µg/g Cr) ≥0.15; urinary IgG4 (µg/g Cr) ≥39.2]. A. Five-year follow-up test on eGFR in type 2 diabetic patients. Data are shown in mean values. B. Five-year follow-up test on eGFR in microalbumin negative patients. Data are shown as mean ± standard deviation. Both positive group showed a significant decline of eGFR compared to control group (p<0.001). C. Five-year follow-up test of microalbumin negative, both positive group. NA: normal albuminuria; MA: microalbuminuria. D. Urinary albumin excretion in microalbuminuria negative patients in each group at the end of 5-year follow-up. Data are shown as mean ± standard deviation. Urinary albumin increased significantly in each group after 5 years compared to the time of start of follow-up (p<0.001). Both positive group exhibited a significantly high urinary albumin excretion compared to control group (p<0.005). However, no difference was observed between groups in the rate of change from the time of start of follow-up to the end of 5-year follow-up.

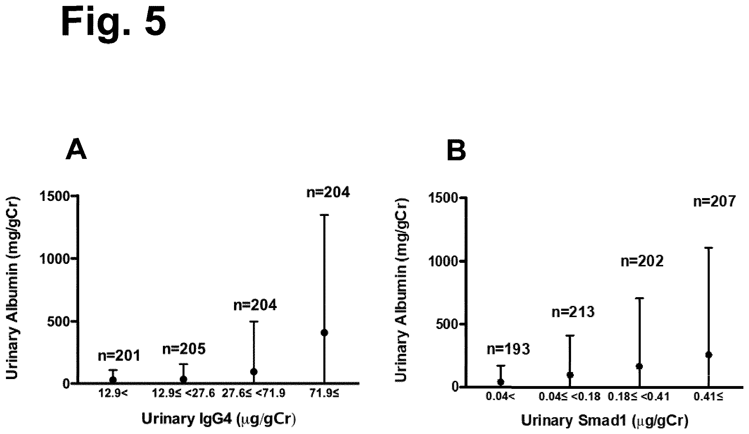

[Fig. 5] Data are shown as mean ± standard deviation. Trends were analyzed by the Jonckheere-Terpstra test. A. Correlation between urinary albumin and urinary IgG4 (quartile analysis). B. Correlation between urinary albumin and urinary Smad1 (quartile analysis).

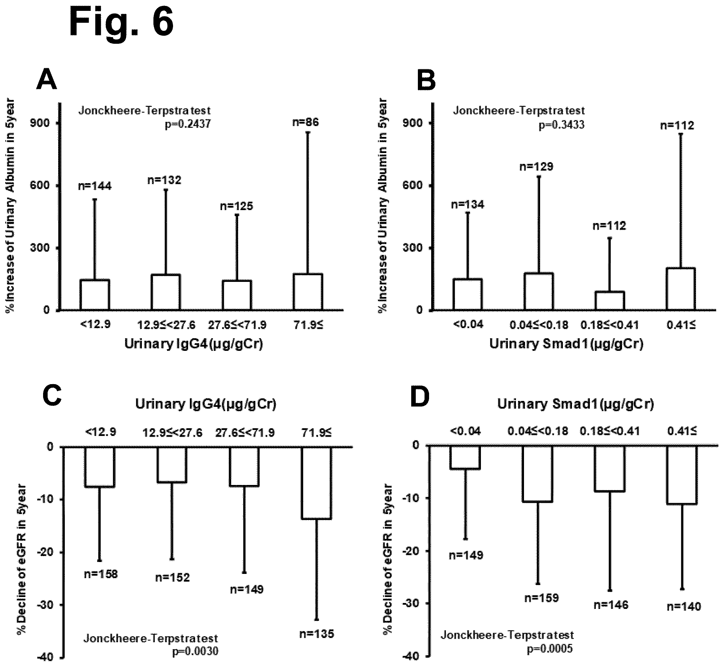

[Fig. 6] Quantile analyses of urinary IgG4 and urinary Smad1 on the progression of eGFR decline at the end of 5-year follow-up as compared to the baseline. Trends were analyzed by the Jonckheere-Terpstra test. A. Progression of albuminuria at the end of 5-year follow-up in quantile analysis of urinary IgG4. B. Progression of albuminuria at the end of 5-year follow-up in quantile analysis of urinary Smad1. C. Progression of eGFR decline at the end of 5-year follow-up in quantile analysis of urinary IgG4. D. Progression of eGFR decline at the end of 5-year follow-up in quantile analysis of urinary Smad1.

### BEST MODES FOR CARRYING OUT THE INVENTION

Hereinbelow, the present invention will be described in detail.

The present invention provides a method of specifically detecting onset of diabetic nephropathy, or early-stage nephropathy, comprising measuring IgG4 in a biological sample derived from a subject.

Diabetic nephropathy is one of microvascular diseases caused by chronic exposure to hyperglycemia. Pathologically, diabetic nephropathy presents hyperplasia of the renal glomerular basement membrane, expansion of the mesangial area and glomerulosclerosis lesions; clinically, diabetic nephropathy presents symptoms such as proteinuria (microalbuminuria), hypertension, edema, etc. Finally, diabetic nephropathy often results in renal failure. Further, in diabetes, abnormalities such as arteriolosclerosis and degeneration/fibrosis in the tubulointerstitium are recognized in tissues other than the glomeruli, worsening glomerular lesions still further. Briefly, a pathological condition, in which proteinuria, hypertension and renal dysfunction gradually progress after a specific period of diabetes duration, can be defined as nephropathy.

Recently, diabetic nephropathy accounts for 40% and more of the original diseases in cases that have turned to a state of end-stage renal failure and are newly introduced into hemodialysis (http://www.jsdt.or.jp/overview_confirm.html) and this ratio has substantially remained on the same level since 2008 (Illustrated Guidebook: Status of Chronic Dialysis Therapy in Japan as of December 31, 2014, published by Japanese Society for Dialysis Therapy on December 1, 2015). Further, prognosis of these patients after the introduction of dialysis is not necessarily good, which is a big issue in medical treatment. Therefore, it has become an important task to elucidate the mechanism of development and progression of diabetic nephropathy and to develop diagnosis and treatment thereof (Japanese Journal of Clinical Medicine vol. 55, 1997 special issue "Diabetes" (1)).

The biological sample may be any biological sample as long as IgG4 is detectable therein. Specific examples of the biological sample which may be used in the invention include renal tissue sections, blood, sera and urine.

Further, Smad1 in a biological sample derived from a subject may be measured.

Still further, it is desirable to measure both IgG4 and Smad1 in a biological sample derived from a subject.

IgG4 is one of the subclasses of IgG (one of immunoglobulins) and accounts for about 3 to 4% of total serum IgG.

Nine Smad proteins including Smad1 to Smad9 have been identified in mammals, and Smad1 is known as a member of the bone morphogenetic protein (BMP) signal transduction pathway. It has also been elucidated that Smad1 transduces TGF-β signals through activin receptor-like kinase 1 (ALK1) to thereby regulate the transcription of target genes in endothelial cells and hematopoietic cells (Goumans MJ. et al., EMBO J., 2002, Apr 2, 21(7), 1743-53).

IgG4 and Smad1 (an optional item) in a biological sample derived from a subject may be measured at nucleic acid level (i.e., expression of mRNA) and/or protein level.

Measurement at nucleic acid level may be performed as follows. Briefly, total RNA is extracted from a biological sample. With an appropriate pair of primers, mRNA levels of IgG4 and Smad1 (an optional item) may be measured by RT-PCR.

Alternatively, after extraction of total RNA from a biological sample, mRNA levels of IgG4 and Smad1 (an optional item) may be measured by northern hybridization using appropriate probes. Probes may be labeled with a suitable substance such as ³²P.

Primers and probes may be designed based on sequence information registered in known nucleic acid data bases.

Measurement at protein level may be performed by immunoassay. For example, using an antibody to IgG4 and an antibody to Smad1 (optional), IgG4 and Smad1 (optional) may be measured by enzyme-linked immunosorbent assay (ELISA), fluorescent antibody method (FA), radioimmunoassay (RIA), fluorescence enzyme immunoassay (FLEIA), chemiluminescence enzyme immunoassay (CLEIA), chemiluminescence immunoassay (CLIA), electro-chemiluminescence immunoassay (ECLIA), immunochromatography (ICA), western blotting (WB), immunoblotting or the like.

Antibodies to IgG4 are commercially available (e.g., mouse monoclonal antibody to human IgG heavy chain). Alternatively, antibodies to IgG4 may be obtained by immunizing an animal with a natural or recombinant IgG4 and preparing a monoclonal antibody by known methods.

Antibodies to Smad1 may be obtained by immunizing an animal with a natural or recombinant Smad1 and preparing a monoclonal antibody by known methods.

Antibodies to IgG4 and antibodies to Smad1 (optional) may be labeled with such substances as fluorescent dye, enzyme or heavy metal (direct method). Alternatively, instead of labeling these antibodies, secondary antibodies specific to these antibodies (primary antibodies) may be used after being labeled with fluorescent dye, enzyme, heavy metal or the like (indirect method). Preferably, antibodies are immobilized on a solid carrier such as test piece or latex particles.

The subject may be a patient with diabetes (especially type 2 diabetes). Preferably, the patient is at an early stage of diabetic nephropathy. If the subject is a diabetic patient not suffering from overt nephropathy, it is possible to diagnose early-stage nephropathy or predict progression into overt nephropathy. Thus, early treatment can be commenced. As a result, it becomes possible to prevent diabetic nephropathy or to prevent or delay the worsening of pathological conditions.

Based on the IgG4 level in a biological sample from a subject, it is possible to judge that the subject has developed diabetic nephropathy or has a high risk of developing diabetic nephropathy. For example, if the biological sample from the subject is urine, it is possible to judge that the subject has developed diabetic nephropathy or has a high risk of developing diabetic nephropathy when the IgG4 level in the subject's urine is 39.2 µg/g Cr or more.

The cutoff value of urinary IgG4 (39.2 µg/g Cr) was calculated as described below.

Logarithmically transformed (base 10) urinary IgG4 was plotted on the horizontal axis and urinary albumin on the vertical axis. As a result, two different distributions were found. In order to perform the hockey stick regression method, 10% point and 75% point of the measured urinary IgG4 values were calculated. Further, the integer parts of the measured urinary IgG4 values were determined, and the obtained values were ranked. Data for points ranging from 10% to 75% were scanned and divided into two for each rank. Regression coefficient for each distribution was determined, and those rank values which gave the largest ratio of regression coefficient were detected. From the distribution of such rank values, logarithmically transformed (base 10) data of urinary IgG4 were scanned again ranging from the minimum to maximum values. From each of the two distributions divided per data, those data which gave the largest ratio of regression coefficient were detected. Using the regression formulas determined for the two distributions (left: urinary albumin = -3.52212 x Log10 (urinary IgG4) + 33.35721, right: urinary albumin = 591.92494 x Log10 (urinary IgG4) - 915.42907), individual urinary albumin values were estimated by repeatedly adding 0.00001 at a time to the maximum value in the left distribution and by repeatedly subtracting 0.00001 at a time from the minimum value in the right distribution. Trial calculations were repeated until the difference satisfies the convergence condition (less than 0.001) to thereby obtain a cutoff value.

The above-described cutoff value may possibly vary when antibodies to IgG4 or the diet, race, type of diabetes (type 1 or 2), age group, etc. of subjects are changed. In such occasions, calibration may, for example, be performed as described below.

When a kit's components (such as antibodies) are changed, measurement is carried out in a certain group of diabetic patients using both of the new and the old kits. Then, correlation analysis of the new and old measured values is performed. If, as a result, a distribution is provided in which a strong positive correlation is recognized and the regression line is judged to be a straight 45° line substantially passing through the origin, the cutoff value will not be changed.

In other cases, a new cutoff value is estimated based on distributions of urinary albumin and urinary IgG4 using regression analysis, etc. in the same manner as described for calculation of the old cutoff value.

When the population of subjects has a major change (e.g., the race is different from the original one), it may be necessary to investigate into the distribution of urinary IgG4 in a group of healthy controls of the race in question and groups of diabetic nephropathy patients of that race, and then examine whether or not the cutoff value obtained from the currently conducted Japanese cohort is valid. If it is necessary to make a change, an appropriate cutoff value may be obtained as described below. Briefly, using urinary IgG4 data from a group of healthy controls of the race in question and groups of diabetic nephropathy patients of that race, various cutoff values are taken to calculate sensitivity and specificity for each cutoff value. ROC curve is constructed by plotting false-positive rate (1 - specificity) on the horizontal axis and true-positive rate (sensitivity) on the vertical axis. Performance of the ROC curve is judged by AUC (area under the curve: taking values from 0 to 1; the area is 1 when complete classification is possible; the area is 0.5 when classification is random; criterion for usual judgment is 0.8 or more) and then an optimal cutoff value is estimated from the ROC curve (ROC analysis).

Further, based on the Smad1 level in a biological sample from a subject, the subject can be judged to have developed diabetic nephropathy or have a high risk of developing diabetic nephropathy. For example, if the biological sample from the subject is urine, the subject can be judged to have developed diabetic nephropathy or have a high risk of developing diabetic nephropathy when Smad1 level in the subject's urine is 0.15 µg/g Cr or more.

The cutoff value of urinary Smad1 (0.15 µg/g Cr) was calculated as described below.

A regression formula was calculated by plotting mesangial area on the horizontal axis and urinary Smad1 on the vertical axis. The mesangial matrix fraction of the normal renal specimens was 5.88 ± 1.30%. Therefore, mesangial matrix fractions >8.5% (corresponding to normal mean + 2 SDs) were considered to represent increased mesangial matrix. The value 8.5 was substituted into the calculated regression formula (urinary Smad = 10.01566 x mesangial matrix + 0.02102). The thus estimated value was taken as a cutoff value.

The above-described cutoff value may vary when antibodies to Smad1 or the diet, race, type of diabetes (type 1 or 2), age group, etc. of subjects are changed. In such occasions, calibration may, for example, be performed as described below.

When a kit's components (such as antibodies) are changed, measurement is carried out in a certain group of diabetic patients using both of the new and the old kits. Then, correlation analysis of new and old measured values is performed. If, as a result, a distribution is provided in which a strong positive correlation is recognized and the regression line is judged to be a straight 45° line substantially passing through the origin, the cutoff value will not be changed.

In other cases, a new cutoff value is determined by the linear regression of mesangial matrix fraction and urinary Smad1 distribution using the value of 8.5% as mesangial matrix fraction, in the same manner as described for calculation of the old cutoff value.

When the population of subjects have a major change (e.g., the race is different from the original one), it may be necessary to investigate into the distribution of urinary Smad1 in a group of healthy controls of the race in question and groups of diabetic nephropathy patients of that race, and then examine whether or not the cutoff value obtained from the currently conducted Japanese cohort is valid. If it is necessary to make a change, an appropriate cutoff value may be obtained as described below. Briefly, using urinary Smad1 data from a group of healthy controls of the race in question and groups of diabetic patients of that race, various cutoff values are taken to calculate sensitivity and specificity for each cutoff value. ROC curve is constructed by plotting false-positive rate (1 - specificity) on the horizontal axis and true-positive rate (sensitivity) on the vertical axis. Performance of the ROC curve is judged by AUC (area under the curve: taking values from 0 to 1; the area is 1 when complete classification is possible; the area is 0.5 when classification is random; criterion for usual judgment is 0.8 or more) and then an optimal cutoff value is estimated from the ROC curve (ROC analysis).

For those subjects who have been judged as having developed diabetic nephropathy or having a high risk of developing diabetic nephropathy according to the above-described measurement, treatment may be started. Major treatment for diabetic nephropathy includes blood glucose control and antihypertensive therapy. While the basics of blood glucose control are low calorie meal and exercise therapy, optionally applicable are the administration of diabetic drugs (e.g., DPP-4 inhibitors, GLP-1 receptor agonists, SGLT2 inhibitors, SU agents, α glucosidase inhibitors, biguanide, quick acting-type insulin secretion promoters, insulin resistance improving agents, or combined drugs) or injection of insulin. Antihypertensive therapy comprises administration of angiotensin-converting enzyme (ACE) inhibitors or angiotensin II receptor antagonists (ARBs), and optionally comprises administration of other types of antihypertensives such as calcium antagonists or diuretics. Further, treatment of diabetic nephropathy using the following drugs has been reported, and these drugs may also be used: growth hormone (GH)/insulin-like growth factor (IGF1) inhibitors (Curr Diabetes Rev (2011) 7(1) 50), Jak/Stat inhibitors (Diabetologia (2016) 59,1624; Diabetes (2009) 58, 469; Nephrol Dial Transplant (2015) 30, iv54), CCL2=MCP1/CCR2 antagonists (Nephrol Dial Transplant (2016) 0,1; Biochem Biophys Res Commun (2007) 360,772; Diabetes Care (2009) 32(3) 465), Nrf2-Keap1 activators (Nephrol Dial Transplant (2014) 29, 119; N Engl J Med (2013) 369 (26) 2492; Nephrol Dial Transplant (2013) 28, 2841; N Engl J Med (2011) 365 (4) 327; Am J Physiol Renal Physiol (2013) 304, F808), Notch1 inhibitors (Nephrol Dial Transplant (2015) 30, iv54), ETAR antagonists (Nephrol Dial Transplant (2015) 30, iv54) and the like.

The method of the present invention may be used for early diagnosis of diabetic nephropathy (especially type 2 diabetes). For example, the method of the present invention is applicable to diagnosis of early-stage nephropathy in diabetes (especially type 2 diabetes) and to prediction of progression into overt nephropathy. Conventionally, measurements of urinary type IV collagen and urinary albumin have been used in diagnosis of diabetic nephropathy. The method of the present invention may possibly replace or complement these methods.

The present invention also provides a method of diagnosis of diabetic nephropathy in a subject. The method of diagnosis of the present invention comprises (a) obtaining a biological sample from the subject, (b) measuring IgG4 level in the biological sample from the subject, and (c) judging that the subject has developed diabetic nephropathy or has a high risk of developing diabetic nephropathy based on the IgG4 level measured.

Further, the present invention also provides a method of diagnosis and treatment of diabetic nephropathy in a subject. The method of diagnosis and treatment of the present invention comprises (a) obtaining a biological sample from the subject, (b) measuring IgG4 level in the biological sample from the subject, (c) judging that the subject has developed diabetic nephropathy or has a high risk of developing diabetic nephropathy based on the IgG4 level measured, and (d) starting treatment of diabetic nephropathy in the subject who is judged as having developed diabetic nephropathy or as having a high risk of developing diabetic nephropathy.

It is possible to judge that a subject has developed diabetic nephropathy or has a high risk of developing diabetic nephropathy when the IgG4 level in a biological sample from the subject is higher than the IgG4 level in a corresponding biological sample from healthy persons who have not developed kidney diseases.

In the judgment, a cutoff value may be used. When the IgG4 level in the biological sample from the subject is higher than a cutoff value, the subject may be judged as having developed diabetic nephropathy or having a high risk of developing diabetic nephropathy.

For example, if the biological sample from the subject is urine, it is possible to judge that the subject has developed diabetic nephropathy or has a high risk of developing diabetic nephropathy when the IgG4 level in the subject's urine is 39.2 µg/g Cr or more.

The method of diagnosis and the method of diagnosis/treatment of the present invention may further comprise measuring Smad1 level in a biological sample derived from a subject and judging that the subject has developed diabetic nephropathy or has a high risk of developing diabetic nephropathy based on the Smad1 level measured.

It is possible to judge that a subject has developed diabetic nephropathy or has a high risk of developing diabetic nephropathy when the Smad1 level in a biological sample from the subject is higher than the Smad1 level in a corresponding biological sample from healthy persons who have not developed kidney diseases.

In the judgment, a cutoff value may be used. When the Smad1 level in the biological sample from the subject is higher than a cutoff value, the subject may be judged as having developed diabetic nephropathy or having a high risk of developing diabetic nephropathy.

For example, if the biological sample from the subject is urine, it is possible to judge that the subject has developed diabetic nephropathy or has a high risk of developing diabetic nephropathy when the Smad1 level in the subject's urine is 0.15 µg/g Cr or more.

Treatment of diabetic nephropathy includes blood glucose control and antihypertensive therapy (as described above).

The subject may be a patient with diabetes (especially type 2 diabetes). Preferably, the subject is at an early stage of diabetic nephropathy.

Further, the present invention also provides a kit for detecting onset or a risk of onset of diabetic nephropathy, comprising a reagent(s) for measuring IgG4. The kit of the present invention may further comprise a reagent(s) for measuring Smad1.

One embodiment of the kit of the present invention is a kit for detecting onset or a risk of onset of diabetic nephropathy by measuring urine samples.

Specific examples of reagents for measuring IgG4 include, but are not limited to, a pair of primers capable of specifically amplifying a specific region of the nucleotide sequence of mRNA encoding IgG4, a probe capable of specifically hybridizing to a part or the whole region of mRNA encoding IgG4, and antibodies to IgG4. These primer pairs and antibodies are as described earlier.

Specific examples of reagents for measuring Smad1 include, but are not limited to, a pair of primers capable of specifically amplifying a specific region of the nucleotide sequence of mRNA encoding Smad1, a probe capable of specifically hybridizing to a part or the whole region of mRNA encoding Smad1, and antibodies to Smad1. These primer pairs and antibodies are as described earlier.

The kit of the present invention may further comprise reverse transcriptases, DNA polymerases, RNase-free water, buffers, control mRNAs, control primer pairs, dNTP mixtures, instructions for using the kit, and the like (if the kit is for measuring the expressions of IgG4 and Smad1 (optional) at nucleic acid level using primer pairs).

Alternatively, the kit of the present invention may further comprise transcription buffers, blocking reagents, rinsing solutions, instructions for using the kit, and the like (if the kit is for measuring the expressions of IgG4 and Smad1 (optional) by western blotting).

In another embodiment, the kit of the present invention may further comprise labeled secondary antibodies, substrates (when secondary antibodies are enzyme-labeled), diluents, quenchers, instructions for using the kit, and the like (if the kit is for measuring the expressions of IgG4 and Smad1 (optional) by ELISA).

In still another embodiment, the kit of the present invention may further comprise color formers, hydrogen peroxide solution, buffers, dyes for counter-staining, instructions for using the kit, and the like (if the kit is for measuring the expressions of IgG4 and Smad1 (optional) by immunohistochemical analysis).

Further, the present invention provides a method of identifying substances effective in preventing and/or treating diabetic nephropathy, comprising judging whether or not a test substance inhibits expression of IgG4. Further, a test substance may be measured as to whether or not it inhibits expression of Smad1.

One embodiment of the above method of the present invention will be described below.

When mesangial cells are cultured in the presence of AGEs (advanced glycation end products), production of BMP4 (bone morphogenetic protein 4) increases. Subsequently, when BMP4 has bound to ALK3 receptor on mesangial cells, phosphorylation of Smad1 occurs. Two molecules of phosphorylated Smad1 and one molecule of Smad4 form a trimer, which moves from the cytoplasm into the nuclei, initiating the transcription of type IV collagen, one of its target genes. Since it is known that this production of type IV collagen is the most critical factor in mesangial matrix expansion, a compound that inhibits this BMP4/ALK3 signal transduction pathway is believed to be a potential prophylactic/therapeutic drug for diabetic nephropathy. This idea is supported by various findings that Smad1 signaling pathway is strongly associated with pathogenesis of diabetes in diabetic animal models.

In STZ-induced diabetic rats, it is recognized that expansion of the mesangial matrix is strongly correlated with urinary Smad1 and it has been confirmed that an increase in urinary Smad1 at an early stage is a predictor for mesangial expansion that will occur later.

Based on these findings, the following test substances, for example, can be evaluated in the manner as described below. Examples of test substances include, but are not limited to, peptides, proteins, non-peptidic compounds, synthetic compounds, fermentation products, cell extracts, plant extracts and animal tissue extracts. These substances may be either novel substances or known substances.

When human mesangial cells are cultured *in vitro* in the presence of AGEs, production of BMP4 protein increases. Human mesangial cells are cultured under AGEs stimulation in the presence or absence of a test substance, followed by measurement of productions of BMP4 protein and type IV collagen protein (a target molecule downstream of BMP4).

Subsequently, a test substance capable of inhibiting BMP4 production or type IV collagen production in vitro is administered to STZ-induced diabetic rats, for example. By measuring urinary Smad1 levels, it is possible to examine the inhibitory effect of the test substance on the progression of diabetic nephropathy.

It should be noted that IgG4 does not exist in either rats or mice. Therefore, measurement of urinary IgG4 may be performed with animals such as dog, pig or monkey that have IgG4. In the case of humans, by measuring urinary IgG4 levels of diabetic patients before and after treatment with drugs such as ARB or ACE inhibitors, one may, for example, be able to evaluate whether or not urinary IgG4 is applicable to prediction of the drug efficacy.

### Examples

Hereinbelow, the present invention will be described in more detail with reference to the following Example.

### [Example 1]

### Methods

### • Patient groups

A group of 871 patients with type 2 diabetes were enrolled at Ikeda Hospital (Amagasaki City, Hyogo Pref., Japan). Prior to the enrollment, informed consent process in writing was carried out. The research protocol conformed to the Declaration of Helsinki and was approved by the Ethical Review Board of Ikeda Hospital. The following patients were excluded: patients with gastrectomy, hepatitis (AST or ALT>100 IU/L or abnormal), and non-diabetic renal diseases (other renal diseases with severe poikilocytes, other test data and clinical symptoms). After obtaining consent, each patient was checked for the presence or absence of diabetic retinopathy. A five-year follow-up study including testing and clinical data gathering was performed on 773 patients (see Fig. 1).

### • Renal biopsy in type 2 diabetic patients

### "Kitasato University renal biopsy specimens"

Renal biopsy was performed at Kitasato University (Sagamihara City, Kanagawa Pref., Japan) in type 2 diabetic patients with normal blood pressure (130/85 mm Hg or below) (3 females, 14 males, age 49.5±11.8, disease duration 14±7 years). These patients did not have overt nephropathy or hematuria, and their serum creatinine levels were 1.2 mg/dl or below. Renal lesions other than definite diabetic nephropathy were not observed in any of the patients. The protocol for this study was approved by the Research Ethics Committee of Kitasato University School of Medicine, and all patients provided written informed consent^{20, 21}.

### "Tokushima University renal biopsy specimens"

Renal biopsy specimens for normal renal tissue were obtained from 10 patients with renal cell carcinoma (3 females, 7 males, age 66.8±13.1). Normal moieties of the kidneys of these patients were analyzed. It was confirmed by oral glucose tolerance test that none of these patients had diabetes. It was also confirmed that none of these patients had proteinuria, renal impairment or hypertension. Pathological findings indicative of renal disorder were not observed in the kidneys of these patients. The protocol of this study was structurally approved by the Research Ethics Committee of Tokushima University (Tokushima City, Tokushima Pref., Japan).

### • Clinical Measurement

Urine samples were centrifuged for 15 min. Supernatants stored at -80°C were rapidly thawed and centrifuged to remove urates or phosphates before use in assays.

Estimated glomerular filtration rate (eGFR) was calculated with a formula targeting Japanese using correction of body surface area²². Urinary albumin was measured by turbidimetric immunoassay. Creatinine was measured by an enzymatic method.

Microalbuminuria was based on two consecutive measurements and urinary albumin-to-creatinine ratio (ACR) >300 mg/g was defined as urinary creatinine while an ACR of 30-300 mg/g Cr was defined as microalbuminuria.
eGFR = 194 x serum creatinine - 1.094 x age - 0.287 x body surface area/1.73 (female eGFR = male eGFR x 0.739)

### • Image analysis of renal biopsy specimens

Renal biopsy specimens were fixed in 10% buffered formalin solution, followed by PAS staining and PASM staining. With respect to the mesangial matrix area, PASM-positive mesangial area was analyzed with Image Processor for Analytical Pathology (IPAP from Sumika Technoservice Corporation). The mesangial area was expressed as percentage of the glomerular total area. Further, glomerulosclerosis, interstitial fibrosis, glomerular surface area and glomerular cell count were measured. For each sample, at least 10 glomeruli were subjected to measurement^{7,13,15,23}.

Tissue specimens for electron microscopy were fixed in 2.5% glutaraldehyde, postfixed in osmium tetraoxide, dehydrated and embedded. Ultrathin sections were examined with an electron microscope (JEOL CX 100, JEOL Ltd.). The thickness of GBM and the GBM fraction in the glomeruli were fixed with grid mesh^{4,21,24-26}. Measurement was impossible for one patient because samples from the patient were unsatisfactory for electron microscopy.

### • Preparation of monoclonal antibodies to human Smad1

Recombinant human Smad1 was isolated by the glutathione S transferase method. Six-week old mice were immunized with the recombinant human Smad1, followed by preparation of monoclonal antibodies according to established methods²⁷. Approximately 3000 hybridoma clones were screened and examined by western blotting and ELISA. SDS-PAGE analysis revealed that the purified protein corresponds to the calculated molecular weight of Smad1. A specific hybridoma clone (Sp125) was then selected.

### • Measurement of urinary Smad1 by ELISA

F(ab)₂ fraction of Sp125 was immobilized on microtiter plates. Urine sample and standard (rhSmad1; 0.2 ng/mL to 5 ng/mL) were reacted with the immobilized fraction, followed by reaction with goat anti-human Smad1 polyclonal antibody (R&D, AF2039). Reaction was then carried out with horseradish peroxidase-conjugated anti-goat IgG antibody. Subsequently, TMB enzyme reaction substrate solution was used for reaction and measurement was conducted at 450 nm.

Urinary Smad1 ELISA was carried out in duplicate, and the mean of three measurements was used. Recovery test was performed with samples of recombinant human Smad1 (concentration: 0.5 ng/mL, 0.2 ng/mL). As a result, the characteristic of assay was confirmed at 111% and 112%, respectively. Minimum detection sensitivity was also examined by the mean ± 3SD method to confirm the sensitivity being 0.2 ng/mL. Reproducibility of assay (CV) was examined by 6 independent assays. CV was 4.06% and reproducibility was confirmed. Urinary Smad1 levels were normalized to the urine Cr concentration.

The specificity of Sp125 antibody was confirmed by western blotting with 5 urine samples and proved as a single band corresponding to Smad1. This band vanished in an absorption test using recombinant human Smad1.

### • Measurement of urinary IgG4 by ELISA

Mouse monoclonal antibody to human IgG heavy chain was immobilized on microtiter plates. Urine sample and standard protein were reacted with human IgG4 full-length protein (1.0 ng/mL, 2000 ng/mL), and then with - mouse-derived anti-human IgG4 antibody conjugated to horseradish peroxidase. Subsequently, TMB enzyme reaction substrate solution was used for reaction and measurement was conducted at 450 nm. Urinary IgG4 ELISA was carried out in duplicate and the mean of three measurements was used.

This urinary IgG4 measurement method showed no cross-reaction with IgG1, IgG2 and IgG3. Recovery test was performed with samples of human IgG4 (concentration: 1000 ng/mL, 20 ng/mL). As a result, the characteristic of assay was confirmed at 103.8% and 118.4%, respectively. Minimum detection sensitivity was 1.0 ng/mL, and reproducibility of assay ranged from 1.0% to 2.1%. The urinary IgG4 concentration was normalized to the urine Cr concentration.

### • Statistical analysis

Data were shown as mean and standard deviation (SD). Trends were analyzed by the Jonckheere-Terpstra test. Values between groups were analyzed by Mann-Whitney U test, one-way ANOVA, Kruskal-Walls test and Chi-square test. Correlation between two variables including pathological parameters and biomarkers was analyzed with Pearson's product-moment correlation coefficient and Spearman's rank-correlation coefficient. The rates of eGFR decline in the follow-up study were analyzed by Wilcoxon's signed rank test within groups and by means of Steele's multiple comparison between groups. As significance level, p<0.05 (two-tailed) was used.

### Results

### • Trend of urinary albumin at different levels of urinary IgG4 and urinary Smad1

In the analysis of urinary IgG4 in type 2 diabetic patients using quartile, albumin excretion was shown to correlate with urinary IgG4 (p<0.0001) (Fig. 5A). Higher urinary Smad1 levels in type 2 diabetic patients correlated with an increase of albumin excretion (p<0.0001) (Fig. 5B).

In the distributions observed in this Example, IgG4 did not exhibit a single distribution. Then, the present inventors performed correlation analysis between log values of urinary IgG4 and albumin values, followed by analysis by hockey stick regression. In this correlation analysis, the inventors prepared a cutoff value of urinary IgG4 which can separate two distributions. The threshold was 1.59341 and the cutoff value was 39.2 µg/g Cr (Fig. 2A). Subsequently, relationships between this cutoff value and various parameters of diabetic nephropathy were analyzed. Compared to the group in which urinary IgG4 was less than 39.2 µg/g Cr (negative group), the group in which urinary IgG4 was 39.2 µg/g Cr or more (positive group) exhibited a significant increase in urinary albumin excretion (p<0.0001, Fig. 2B) and a significant decline in eGFR (p<0.0001, Fig. 2C), whereas no difference was observed with respect to HbA1c (Fig. 2D). Further, in the comparison between these two groups, a significant difference was observed in diabetes duration, the presence of retinopathy, the presence of neurosis, and urinary Smad1 (Table 1). The above-described results show that the cutoff value of urinary IgG4 is clinically very useful.

Since it has been suggested that measurement of total urinary IgG excretion is useful as an indicator for early diagnosis of diabetic nephropathy, the present inventors also examined the relationship between total urinary IgG and urinary albumin excretion. As a result, a significant positive correlation was observed (p<0.0001, Fig. 2E), but a cutoff value as seen for urinary IgG4 could not be found.

**(Table. 1) Difference in parameters on the cutoff value of urinary IgG4**

| | | | |
|---|---|---|---|
| Urinary IgG4 (*µ*g/g Cr) | <39.2 | 39.2≦ | |
| n | 483 | 331 | |
| Gender (% male) | 64.0 | 66.5 | p=0.4644 |
| Age | 62.1 ± 10.3 | 65.7 ± 9.9 | p < 0.0001 |
| Diabetes duration (month) | 157.9 ± 100.2 | 191.2 ± 107.7 | p < 0.0001 |
| Retinopathy (%) | 30.0 | 43.8 | p < 0.0001 |
| BMI (kg/m²) | 23.67 ± 3.41 | 23.86 ± 3.58 | p = 0.4539 |
| Mean Blood Pressure (mmHg) | 93.64 ± 10.10 | 94.84 ± 11.17 | p = 0.1207 |
| Urinary Smad1 (mg/gCr) | 0.319 ± 0.661 | 0.398 ± 0.570 | p < 0.0001 |
| eGFR (mL/min) | 73.49 ± 20.29 | 66.46 ± 23.24 | p < 0.0001 |
| eGFR (mL/min/1.73m²) | 76.53 ± 18.13 | 69.71 ± 22.76 | p < 0.0001 |
| Serum Albumin (g/dl) | 4.29 ± 0.28 | 4.21 ± 0.33 | p < 0.01 |

| Medication (%) | | | |
|---|---|---|---|
| Anti-hypertensive drugs | 37.3 | 48.3 | p < 0.002 |
| (RAS inhibitors) | (23.8) | (36.3) | (p < 0.0001) |
| Statin | 28.4 | 31.1 | p = 0.3974 |
| Uric Acid lowering drugs | 4.3 | 8.5 | p < 0.05 |

### • Renal tissue changes and urinary IgG4 and urinary Smad1

Correlations between histopathological parameters and urinary biomarkers are shown in Table 2.

**(Table 2) Correlations between pathological parameters and biomarkers**

| | Urinary IgG4 (*µ*g/g Cr) | Urinary Smad1 (*µ*g/g Cr) | Urinary Albumin (mg/g Cr) |
|---|---|---|---|
| Mesangial Matrix Fraction (%) | ρ = 0.048 | r = 0.564 | ρ = 0.060 |
| | p = 0.8554 | p = 0.0184 | p = 0.8188 |
| GBM Thickness (nm) | ρ =- 0.053 | r = 0.468 | ρ = -0.362 |
| | p = 0.8456 | p = 0.0673 | p = 0.1686 |
| Sv (PGBM/glom) | ρ = 0.847 | r = -0.175 | ρ = 0.226 |
| | p < 0.0001 | p = 0.5170 | p = 0.3990 |
| Global Sclerosis (%) | ρ = 0.278 | r = 0.150 | ρ = 0.382 |
| | p = 0.2795 | p = 0.5658 | p = 0.1297 |
| Interstitial Fibrosis (%) | ρ = 0.049 | r = 0.380 | ρ = -0.186 |
| | p = 0.8518 | p = 0.1329 | p = 0.4741 |
| Glomerular Surface Area (mm²) | ρ = 0.159 | r = 0.354 | ρ = 0.184 |
| | p = 0.5414 | p = 0.1633 | p = 0.4800 |
| Glomerular Cell Number (n) | ρ = -0.002 | r = -0.172 | ρ =0.512 |
| | p = 0.9926 | p = 0.5091 | p = 0.0355 |

| | | | |
|---|---|---|---|
| GBM: glomerular basement membrane | | | |

In this small-sized cohort, urinary albumin showed a significant correlation with glomerular cell number (p<0.05, Table 2). Increase in urinary IgG4 showed a significant positive correlation with an increase in GBM fraction occupying the gromeluli (p<0.0001, Fig. 3A). On the other hand, examination of the relation between total urinary IgG and GBM fraction occupying the gromeluli revealed no correlation unlike the case of IgG4 (Fig. 3B). These results show that urinary IgG4 rather than total urinary IgG is a good biomarker that reflects the thickening of the glomerular basement membrane.

Urinary Smad1 showed a significant correlation with the mesangial matrix (p<0.05, Fig. 3C). The mesangial matrix fraction in normal renal tissue was 5.88±1.30%. Therefore, mesangial matrix expansion was defined as 8.5% that corresponds to normal mean + 2SD. The cutoff value of urinary Smad1 was defined as 0.15 (µg/g Cr) based on the mesangial matrix expansion (Fig. 3C).

### • Baseline data of 5-year follow-up study

The present inventors compared the follow-up data on four different groups (control group; urinary Smad1 positive group; urinary IgG4 positive group; and urinary Smad1/IgG4 both positive group) according to the above-described cutoff values.

In the comparison of these four groups, correlations were observed in diabetes duration, the presence or absence of retinopathy, urinary albumin, the presence or absence of proteinuria, decline in eGFR, use or non-use of antihypertensives (especially RAS inhibitors) and use or non-use of uric acid lowering drugs; however, no significant difference was observed in BMI, blood pressure and HbA1c (Table 3).

**(Table 3) Baseline data of follow-up study patients**

| | Control | U-Smad1 | U-IgG4 | Both | |
|---|---|---|---|---|---|
| Urinary Smad1 (*µ*g/g Cr) | < 0.15 | 0.15 ≦ | < 0.15 | 0.15 ≦ | |
| Urinary IgG4 (*µ*g/g Cr) | < 39.2 | < 39.2 | 39.2 ≦ | 39.2 ≦ | |
| n | 243 | 240 | 115 | 216 | |
| Gender (% male) | 71.2 | 56.7 | 67.8 | 65.7 | p = 0.008 |
| Age | 61.6 ± 10.7 | 62.7 ± 10.0 | 64.3 ± 10.1 | 66.4 ± 9.7 | p < 0.0001 |
| Diabetes duration (month) | 148.9 ± 96.0 | 166.9 ± 103.7 | 172.9 ± 99.2 | 200.9 ± 111.0 | p < 0.0001 |
| Presence of retinopathy (%) | 25.5 | 34.6 | 40.0 | 45.8 | p < 0.0001 |
| BMI (kg/m²) | 23.85 ± 3.39 | 23.49 ± 3.43 | 23.95 ± 3.86 | 23.81 ± 3.43 | p = 0.5764 |
| Mean Blood Pressure (mmHg) | 93.54 ± 9.88 | 93.75 ± 10.34 | 93.84 ± 10.79 | 95.36 ± 11.35 | p = 0.2503 |
| Urinary Albumin (mg/g Cr) | 27.70 ± 92.03 | 42.71 ± 140.86 | 107.63 ± 225.18 | 400.55 ± 970.15 | p< 0.0001 |
| eGFR (mL/min) | 74.28 ± 19.27 | 72.70 ± 21.27 | 70.51 ± 21.92 | 64.31 ± 23.68 | p< 0.0001 |
| eGFR (mL/min/1.73 m²) | 76.25 ± 17.00 | 76.82 ± 19.24 | 73.30 ± 21.19 | 67.80 ± 23.37 | p< 0.0001 |
| HbA1c (%) | 7.33 ± 1.00 | 7.53 ± 1.12 | 7.60 ± 1.06 | 7.55 ± 1.14 | P = 0.0878 |

| Medication (%) | | | | | |
|---|---|---|---|---|---|
| Anti-hypertensive drugs | 35.8 | 38.8 | 45.2 | 50.0 | p< 0.02 |
| (RAS inhibitors) | (21.8) | (25.8) | (33.0) | (38.0) | (p< 0.001) |
| Statin | 23.9 | 32.9 | 30.4 | 31.5 | p = 0.1380 |
| Uric Acid lowering drugs | 3.3 | 5.4 | 5.2 | 10.2 | p< 0.02 |

Gender, retinopathy, proteinuria and medication are shown in percentage. Age, diabetes duration, BMI, mean blood pressure, urinary albumin, eGFR, eGFR/1.73 m³ and HbA1c are shown as mean ± standard deviation.

### • Five-year follow-up study of type 2 diabetic patients

Fig. 4A shows the results of 5-year follow-up of eGFR decline in four different groups (control group; urinary Smad1 positive group; urinary IgG4 positive group; and urinary Smad1/IgG4 both positive group). In the control group, urinary Smad1 positive group and urinary IgG4 positive group, almost parallel eGFR decline trends were observed. In these three groups, a significant eGFR decline was observed at year 4 and thereafter, as compared to the time of baseline. The eGFR decline in the control group was -4.44±9.78 mL/min/ in five years. In contrast, in urinary Smad1/IgG4 both positive group, a significant eGFR decline began to be observed from one year after the start of the follow-up, as compared to the time of baseline; and a significant eGFR decline was observed in any year of the 5-year follow-up. These results, showing that when both urinary Smad1 and urinary IgG4 have become positive, a significant eGFR decline is very likely to occur one year later, suggest that if both biomarkers become positive, treatment should be started immediately.

As described above, urinary IgG4 and urinary Smad1 clearly correlated with early-stage kidney-specific structural changes in type 2 diabetic patients, so the inventors analyzed those patients who had microalbuminuria at the start of the follow-up. As a result, a definite eGFR decline was clearly seen in IgG4/Smad1 both positive group during the 5-year follow-up period (Fig. 4B).

Further, those patients who had microalbuminuria in IgG4/Smad1 both positive group at the start of the follow-up showed a significant eGFR decline in five years, as compared to those patients with normal albuminuria (Fig. 4C). This finding was not observed in the other groups. Further, although IgG4/Smad1 both positive group showed a significantly high urinary albumin excretion compared to the control group (p<0.005), no inter-group difference was observed with respect to rate of increase in urinary albumin at 5 years after the start of the follow-up (Fig. 4D).

Quartile analysis of urinary IgG4 and urinary Smad1 revealed that high levels of urinary IgG4 or urinary Smad1 exhibit a greater eGFR decline during the 5 year follow-up period (Fig. 6C, D). On the other hand, urinary IgG4 and urinary Smad1 did not correlate with the progression of albuminuria in the quartile analysis (Fig. 6A, B).

### Discussion

Although urinary albumin is used widely as a biomarker for the progression of early-stage nephropathy²⁸, it is not specific to early-stage nephropathy in diabetic nephropathy. Besides, there are no high-sensitivity and specific biomarkers that reflect pathological tissues characteristic of early-stage nephropathy⁶. The present inventors have proved for the first time that not urinary albumin or total urinary IgG⁶ but both urinary IgG4 and urinary Smad1 reflect specific pathological findings at early stages of diabetic nephropathy⁶. Further, it has become clear that urinary Smad1/IgG4 both positive group not suffering from overt nephropathy at the start of follow-up has a stronger risk with respect to the progression into overt nephropathy and eGFR decline in the subsequent 5 years, as exemplified by this group showing a significant eGFR decline one year after the start of follow-up. In brief, the urinary Smad1/IgG4 both positive state is a biomarker that strongly reflects the findings of specific tissue lesions in nephropathy and which is capable of specifically predicting the risk of diabetic nephropathy.

In modern society, diabetes has become the major cause of CKD, and 20-30% of diabetic patients will contract CKD. Regardless of the use of hypoglycemic drugs and RAS inhibitors, frequency of onset of diabetic nephropathy is increasing²⁹. One of the reasons for this is the inability to diagnose diabetic nephropathy correctly at early stages, because lesions of overt diabetic nephropathy develop and progress before the appearance of current clinical indicators such as albuminuria. Actually, in Japanese type 2 diabetic patients, a large number of long-standing diabetic patients with normal albuminuria showed changes of tissue lesions^{9,10}, though no significant differences were observed in normal albuminuria patients and microalbuminuria patients with respect to the degrees of mesangial matrix expansion and GBM thickening²¹. Therefore, it is necessary to discover a biomarker that takes the place of albumin and suggests renal lesions of early-stage nephropathy.

In the present invention, the inventors have demonstrated that elevated urinary IgG4 and Smad1 are correlated with GBM lesion (Sv: rate of GBM in the glomeruli) and lesion of mesangial matrix expansion, respectively, both lesions being specific to early-stage nephropathy in diabetes. It should be emphasized here that these lesions could not be detected with sustained microalbuminuria or total urinary IgG. In brief, this significant correlation of urinary IgG4 and Smad1 with renal structural changes proves that these markers are highly sensitive biomarkers capable of detecting renal lesions at early stages of nephropathy when the relationship between urinary albumin and renal tissue change is obscure.

The correlation between the urinary Smad1/IgG4 both positive state and GFR decline is believed to reflect the correlation between macroalbuminuria and a worsened state of lesions. Similarly, the findings about the relationships between the urinary Smad1/IgG4 both positive state and diabetes duration and diabetic retinopathy are also believed to suggest the relationship between the Smad1/IgG4 both positive state and overt nephropathy. Recently, it has been reported that GBM thickening is one of risk factors for progression into overt nephropathy or end-stage renal failure in type 1 diabetic patients³¹. Besides, although no proof has been established for the causal relationship of renal structure and function at early stages of type 2 diabetes²¹, it has been reported that GBM thickening and mesangial expansion predict an increase of albuminuria after six years in type 2 diabetic patients³². These reports are believed to support that the urinary Smad1/IgG4 both positive state is an excellent biomarker capable of specifically predicting a risk for the worsening of diabetic nephropathy.

As described so far, the present inventors have proved that Smad1 and IgG4 are important molecules holding the key to the progression of diabetic nephropathy, and have revealed that these markers are useful markers that reflect renal structural changes and that are capable of predicting the onset of diabetic nephropathy. The findings obtained in the present invention, that urinary Smad1 and urinary IgG4 are both predictors for the risk of diabetic nephropathy and targets of treatment at the same time, will be a contributing factor to the initiation of translational research in which discoveries from basic research are applied in clinical practice.

### References

1. Krolewski M, Eggers PW, Warram JH. Magnitude of end-stage renal disease in IDDM: a 35 year follow-up study. Kidney Int 1996; 50:2041-2046.
2. Bojestig M, Arnqvist HJ, Hermansson G, Karlberg BE, Ludvigsson J. Declining incidence of nephropathy in insulin-dependent diabetes mellitus. N Engl J Med 1994; 330:15-18.
3. Parving H-H, Mauer M, Ritz E. Diabetic nephropathy. In: Brenner BM, ed. Brenner & Rector's The Kidney. 7th ed. Philadelphia, USA. Saunders, Elsevier's Health Sciences Department, 2004: 1777-1818.
4. Mauer SM, Steffes MW, Ellis EN, Sutherland DE, Brown DM, Goetz FC. Structural-functional relationships in diabetic nephropathy. J Clin Invest 1984; 74:1143-1155.
5. Kriz W, Lehir M. Pathways to nephron loss starting from glomerular disease- Insights from animal models. Kidney Int 2005; 67:404-419.
6. Caramori ML, Kim Y, Huang C, et al. Cellular basis of diabetic nephropathy: 1. Study design and renal structural-functional relationships in patients with long-standing type 1 diabetes. Diabetes 2002; 51:506-13.
7. Doi T, Vlassara H, Kirstein M, Yamada Y, Striker GE, Striker LJ. Receptor-specific increase in extracellular matrix production in mouse mesangial cells by advanced glycosylation endproducts is mediated via platelet derived growth factor. Proc Natl Acad Sci USA 1992; 89:2873-2877.
8. Abe H, Matsubara T, Iehara N, et al. Type IV collagen is transcriptionally regulated by Smad1 under advanced glycation end product (AGE) stimulation. J Biol Chem 2004; 279:14201-14206.
9. Matsubara T, Abe H, Arai H, et al. Expression of Smad1 is directly associated with glomerulosclerosis in diabetic nephropathy. Lab Invest 2006; 86:357-68.
10. Mima A, Arai H, Matsubara T, et al. Urinary Smad1 is a novel marker to predict later onset of mesangial matrix expansion in diabetic nephropathy. Diabetes 2008; 57:1712-22.
11. Mima A, Matsubara T, Arai H, et al. Angiotensin II-dependent Src and Smad1 signaling pathway is crucial for the development of diabetic nephropathy. Lab Invest 2006; 86:927-939.
12. Ohashi S, Abe H, Takahashi T, et al. Advanced glycation end products increase collagen-specific chaperone protein in mouse diabetic nephropathy. J Biol Chem 2004; 279:19816-23.
13. Takahashi T, Abe H, Arai H, et al. Activation of STAT3/Smad1 is a key signaling pathway for progression to glomerulosclerosis in experimental glomerulonephritis. J Biol Chem 2005; 280:7100-7106.
14. Tominaga T, Abe H, Ueda O, et al. Activation of BMP4 signaling leads to glomerulosclerosis that mimics diabetic nephropathy. J Biol Chem 2011; 286:20109-20116.
15. Kishi S, Abe H, Akiyama H, et al. Sox9 protein induces a chondrogenic phenotype of mesangial cells and contributes to advanced diabetic nephropathy. J Biol Chem 2011; 286: 32162-32169.
16. Abe H, Tominaga T, Matsubara T, et al. Scleraxis modulates bone morphogenetic protein 4 (BMP4)-Smad1-smooth muscle α-actin (SMA) signal transduction in diabetic nephropathy. J Biol Chem 2012; 287:20430-42.
17. Matsubara T, Araki M, Abe H, et al. Bone morphogenetic protein 4 and Smad1 mediate extracellular matrix production in the development of diabetic nephropathy. Diabetes 2015, 64(8):2978-90.
18. Deckert T, Feldt-Rasmussen B, Djurup R, et al. Glomerular size and charge selectivity in insulin-dependent diabetes mellitus. Kidney Int 1988; 33: 101-106.
19. Melvin T, Kim Y, Michael AF. Selective binding IgG4 and other negatively charged plasma proteins in normal and diabetic human kidneys. Am J Pathol 1984; 115: 443-446.
20. Moriya T, Tsuchiya A, Okizaki S, et al. Glomerular hyperfiltration/ increased glomerular filtration surface are associated with renal function decline in normo- and microalbuminuric type 2 diabetes. Kidney Int 2012; 81: 486-493.
21. Moriya T, Moriya R, Yajima Y, et al. Urinary albumin as an indicator of diabetic nephropathy lesions in Japanese type 2 diabetic patients. Nephron 2002; 91:292-299.
22. Matsuo S, Imai E, Horio M, et al. Revised equations for estimated GFR from serum creatinine in Japan. Am J Kidney Dis 53:982-92. 2009
23. Yamamoto Y, Kato I, Doi T, et al. Development and prevention of advanced diabetic nephropathy in RAGE-overexpressing mice. J Clin Invest 2001; 108: 261-268
24. Chavers BM, Bilous RW, Ellis EN, et al. Glomerular lesions and urinary albumin excretion in type I diabetes without overt proteinuria. N Engl J Med 1989; 320:966-970.
25. Fioretto P, Steffes MW, Mauer M. Glomerular structure in nonproteinuric IDDM patients with various levels of albuminuria. Diabetes 1994; 43:1358-1364.
26. Jensen EB, Gundersen HJ, Osterby R. Determination of membrane thickness distribution from orthogonal intercepts. J Microsc 1979; 115: 19-33.
27. Uno S, Kinoshita Y, Azuma Y, et al. Antitumor activity of a monoclonal antibody against CD47 in xenograft models of human leukemia. Oncol Rep 2007; 17:1189-1194
28. Caramori ML, Fioretto P, Mauer M. The need for early predictors of diabetic nephropathy risk: is albumin excretion rate sufficient? Diabetes 2000; 49:1399-408.
29. de Boer IH, Rue TC, Hall YN, et al. Temporal trends in the prevalence of diabetic kidney disease in the United States. JAMA 2011; 305:2532-9.
30. Adler AI, Stevens RJ, Manley SE, et al. Development and progression of nephropathy in type 2 diabetes: the United Kingdom Prospective Diabetes Study (UKPDS 64). Kidney Int 2003; 63:225-32.
31. Caramori ML, Parks A, Mauer M. Renal lesions predict progression of diabetic nephropathy in type 1 diabetes. J Am Soc Nephrol 2013; 24: 1175-81.
32. Moriya T, Tanaka K, Hosaka T, et al. Renal structure as an indicator for development of albuminuria in normo- and microalbuminuric type 2 diabetic patients. Diabetes Res Clin Pract 2008; 82:298-304.

All publications, patents and patent applications cited herein are incorporated herein by reference in their entirety.

### INDUSTRIAL APPLICABILITY

The present invention is applicable to early diagnosis of diabetic nephropathy.

## Claims

1. A method of detecting onset or a risk of onset of diabetic nephropathy, comprising measuring IgG4 in a biological sample derived from a subject.

2. The method of claim 1, further comprising measuring Smad1 in the biological sample derived from the subject.

3. The method of claim 1 or 2, wherein the biological sample is urine.

4. The method of any one of claims 1 to 3, which is used for early diagnosis of diabetic nephropathy.

5. A kit for detecting onset or a risk of onset of diabetic nephropathy, comprising a reagent(s) for measuring IgG4.

6. The kit of claim 5, further comprising a reagent(s) for measuring Smad1.

7. A method of diagnosing diabetic nephropathy in a subject, comprising:
a. obtaining a biological sample from the subject,
b. measuring IgG4 level in the biological sample from the subject, and
c. judging that the subject has developed diabetic nephropathy or has a high risk of developing diabetic nephropathy based on the IgG4 level measured.

8. A method of diagnosis and treatment of diabetic nephropathy in a subject, comprising:
a. obtaining a biological sample from the subject,
b. measuring IgG4 level in the biological ample from the subject,
c. judging that the subject has developed diabetic nephropathy or has a high risk of developing diabetic nephropathy based on the IgG4 level measured, and
d. starting treatment of diabetic nephropathy in the subject who is judged as having developed diabetic nephropathy or as having a high risk of developing diabetic nephropathy.

9. The method of claim 7 or 8, wherein the subject is judged as having developed diabetic nephropathy or as having a high risk of developing diabetic nephropathy when the IgG4 level in the biological sample from the subject is higher than the IgG4 level in a corresponding biological sample from healthy persons who have not developed diabetic nephropathy.

10. The method of claim 7 or 8, wherein the subject is judged as having developed diabetic nephropathy or as having a high risk of developing diabetic nephropathy when the IgG4 level in the biological sample from the subject is higher than a cutoff value.

11. The method of claim 7 or 8, wherein the biological sample from the subject is urine and the subject is judged as having developed diabetic nephropathy or as having a high risk of developing diabetic nephropathy when the IgG4 level in the subject's urine is 39.2 µg/g Cr or more.

12. The method of claim 7 or 8, further comprising measuring Smad1 level in the biological sample from the subject and judging that the subject has developed diabetic nephropathy or has a high risk of developing diabetic nephropathy based on the Smad1 level measured.

13. The method of claim 12, wherein the subject is judged as having developed diabetic nephropathy or as having a high risk of developing diabetic nephropathy when the Smad1 level in the biological sample from the subject is higher than the Smad1 level in a corresponding biological sample from healthy persons who have not developed diabetic nephropathy.

14. The method of claim 12, wherein the subject is judged as having developed diabetic nephropathy or as having a high risk of developing diabetic nephropathy when the Smad1 level in the biological sample from the subject is higher than a cutoff value.

15. The method of claim 14, wherein the biological sample from the subject is urine and the subject is judged as having developed diabetic nephropathy or as having a high risk of developing diabetic nephropathy when the Smad1 level in the subject's urine is 0.15 µg/g Cr or more.

16. The method of claim 8, wherein the treatment of diabetic nephropathy is glycemic control and/or antihypertensive therapy.

17. The method of claim 7 or 8, wherein the subject is at an early stage of diabetic nephropathy.

18. A method of identifying substances effective in preventing and/or treating diabetic nephropathy, comprising judging whether or not a test substance inhibits expression of IgG4.

19. The method of claim 2, wherein IgG4 and Smad1 levels are measured by immunoassay.

20. The kit of claim 6 for detecting onset or a risk of onset of diabetic nephropathy by measuring urine samples.
